# EUROPÄISCHE PATENTSCHRIFT

(11) **EP 4 494 609 B1**
(45) Veröffentlichungstag und Bekanntmachung des Hinweises auf die Patenterteilung: **22.07.2026**
(21) Anmeldenummer: 24186585.6
(22) Anmeldetag: 04.07.2024
(51) Int. Cl.: A61F 2/38, A61F 2/30

(54) **ORTHOPÄDISCHE KNIEGELENKPROTHESE**
ORTHOPAEDIC KNEE JOINT PROSTHESIS
PROTHÈSE ORTHOPÉDIQUE DE L'ARTICULATION DU GENOU

(30) Priorität: 17.07.2023 DE 102023118837
(43) Veröffentlichungstag der Anmeldung: 22.01.2025
(73) Patentinhaber: Aesculap AG, 78532 Tuttlingen (DE)
(72) Erfinder: Bollinger, Arthur, 78532 Tuttlingen (DE); Stoll, Stephanie, 78667 Villingendorf (DE); Gerlitz, Jana, 91154 Roth (DE)
(74) Vertreter: DREISS Patentanwälte PartG mbB

(56) Entgegenhaltungen:
- US-A1- 2016 235 955
- US-A1- 2019 029 833
- US-A1- 2020 246 151
- US-A1- 2022 096 242
- US-B2- 9 820 858

## Beschreibung

Die Erfindung betrifft ein femorales Implantatteil für eine orthopädische Kniegelenkprothese, wobei das femorale Implantatteil wenigstens einen einem Knochen zugewandten und im implantierten Zustand mit dem Knochen in Kontakt stehenden Oberflächenbereich aufweist. Die Erfindung betrifft auch ein tiborales Implantatteil für eine orthopädische Kniegelenkprothese, und eine orthopädische Kniegelenkprothese, sowie ein Herstellungsverfahren zum Herstellen der Implantatteile **bzw.** der orthopädischen Kniegelenkprothese. Femorale und tiborale Implantatteile und derartige Kniegelenkprothesen sind aus dem Stand der Technik bekannt. Für solche Implantate verwendeten Materialien bestehen im Wesentlichen aus Kobalt-Chrom **bzw.** Kobalt-Chrom -MolybdänLegierungen (Co-Cr-Mo) oder keramischen Werkstoffen. Die Prothese wird in der Regel mit Knochenzement am Knochen oder durch Einwachsen von Knochen direkt am Knochen befestigt. Metall- oder Keramikmaterialien verursachen einige unerwünschte klinische Probleme. Aufgrund der vergleichsweise guten Festigkeit und Stabilität und der guten biologischen Verträglichkeit von Polyetheretherketon (PEEK) Polymerwerkstoffen, ist es beispielsweise auch bekannt, Implantate aus Polyetheretherketon (PEEK) Polymerwerkstoffen herzustellen.

Aus US 2020/0246151 A1 ist eine Femurkomponente bekannt, mit einer Innenfläche zur Befestigung an einem präparierten distalen Femur. Die Innenfläche umfasst Abschnitte, die mit zueinander parallelen und orthogonalen Rippen und Nuten versehen sind. Diese sind so angeordnet, dass sie eine erhöhte oder verringerte Steifigkeit der Femurkomponente bei Auskragung um die Gelenkachse im Vergleich zur Steifigkeit bei Auskragung um eine Querachse bewirken.

US 2016/235955 A1 zeigt eine Rippenstruktur bei einem Spacer der bei bakteriell infizierten Endoprothesen in Hüfte, Knie oder Schulter vor Einsatz einer neuen Endoprothese implantiert wird, der beispielsweise entsprechend Medikamente enthält und abgibt und nur temperorär (bspw. für einige Wochen) im Gelenk verbleibt, um die Infektion zu beheben. Der Spacer umfasst Verstärkungselemente gegen mechanische Belastung und zur Aufnahme von Knochenzement beschrieben in Form in Form von zueinander parallelen und orthogonalen Rippe.

Aufgrund der vergleichsweise hohen Stoßbelastung, der sehr komplexen Oberflächenform des Kniegelenks und des relativ geringen Anpassungsgrads ist der Druck auf die Oberfläche des Kniegelenks (10 bis 20 MPa) vergleichsweise hoch, beispielswiese viel höher als in einem Hüftgelenk (2 bis 5 MPa). Diese relativ hohen mechanischen Umgebungsbedingungen und Anforderungen führen zu einem erheblichen Verschleiß eines solchen Implantats.

Der vorliegenden Erfindung liegt die Aufgabe zugrunde, ein femorales Implantatteil für eine orthopädische Kniegelenkprothese bzw. eine orthopädische Kniegelenkprothese bereitzustellen, mit der die genannten Nachteile überwunden werden können.

Diese Aufgabe wird gelöst durch ein femorales Implantatteil gemäß Anspruch 1.

Eine Ausführungsform betrifft ein femorales Implantatteil für eine orthopädische Kniegelenkprothese, wobei das femorale Implantatteil wenigstens einen dem Knochen zugewandten und im implantierten Zustand mit dem Knochen in Kontakt stehenden Oberflächenbereich aufweist, wobei das femorale Implantatteil wenigstens teilweise, zumindest der mit dem Knochen in Kontakt stehende Oberflächenbereich, aus einem Kunststoff, insbesondere PolyArylEtherKetone, (PAEK), insbesondere Polyetheretherketon (PEEK) oder Polyetheretherketon, (PEEK),-Verbundwerkstoff, gefertigt ist, und wobei das femorale Implantatteil eine dreidimensionale Oberflächenstruktur bei dem dem Knochen zugewandten Oberflächenbereich aufweist, und die dreidimensionale Oberflächenstruktur wenigstens eine Rippenstruktur umfasst, wobei wenigstens ein sich auf einer Oberfläche des Oberflächenbereichs in einer Ebene erstreckender erster Rippenabschnitt oder eine sich auf der Oberfläche des Oberflächenbereichs in der Ebene erstreckende erste Rippe der Rippenstruktur und ein sich auf der Oberfläche des Oberflächenbereichs in der Ebene erstreckender zweiter Rippenabschnitt oder eine sich auf der Oberfläche des Oberflächenbereichs in der Ebene erstreckende zweite Rippe der Rippenstruktur in der Ebene zumindest abschnittsweise in voneinander verschiedene Richtungen verlaufen.

Die Rippenstruktur ist also auf einer Oberfläche des Oberflächenbereichs ausgebildet, und bildet so die dreidimensionale Oberflächenstruktur. Die Rippenstruktur umfasst wenigstens zwei oder mehr Rippen und/oder wenigstens zwei oder mehr Rippenabschnitte. Diese Rippen bzw. Rippenabschnitte sind auf der Oberfläche des Oberflächenbereichs angeordnet bzw. darauf ausgebildet. Die Rippen bzw. Rippenabschnitte ragen somit ausgehend von der Oberfläche des Oberflächenbereichs davon ab bzw. stehen ausgehend von der Oberfläche hervor. Unter einer Rippenstruktur wird eine ausgehend von der Oberfläche des Oberflächenbereichs ausgewölbte stegförmige Struktur verstanden.

Die Rippen bzw. Rippenabschnitte ragen makroskopisch über wenigstens 0,5 mm und insbesondere bis 3 mm von einer Oberfläche des jeweiligen Oberflächenbereichs des Implantatteils hervor. In der Ebene, in der sich die Rippen bzw. Rippenabschnitte erstrecken, weisen diese eine Breite von wenigstens 0,5 mm und insbesondere bis 3 mm auf. Die Rippen bzw. Rippenabschnitte weisen in der Ebene eine beliebige Länge, insbesondere von wenigstens 1 mm auf. Eine maximale Länge wird beispielsweise durch eine konkrete Anordnung mehrerer Rippen bzw. Rippenabschnitte und/oder durch eine Erstreckung des Oberflächenbereichs begrenzt.

Zwei Rippenabschnitte könne einer gemeinsamen Rippe oder zwei verschiedenen Rippen zugeordnet sein. Eine jeweilige Rippe und auch ein jeweiliger Rippenabschnitt können an sich in der Ebene beliebig verlaufen, insbesondere geradlinig aber auch abgewinkelt oder gekrümmt.

Die auf der Oberfläche vorgesehene Rippenstruktur erhöht die Stabilität des femoralen Implantats. Die Verbesserung der Stabilität ist deswegen vorteilhaft, da ein Implantat aus Kunststoff, insbesondere Polyetheretherketon (PEEK) oder Polyetheretherketon, (PEEK),-Verbundwerkstoff, geringere Festigkeitswerte aufweist als ein vergleichbares Implantat aus Metall, beispielsweise aus einer Kobalt-Chrom-Legierung.

Grundsätzlich kann das femorale Implantatteil zementfrei oder unter Verwendung von Knochenzement mit dem Knochen verankert werden.

Zudem kann die vorgesehene Rippenstruktur vorteilhaft sein, um eine zementierte Verankerung des Implantats mit dem Knochen zu verbessern.

Es ist beispielsweise vorgesehen, dass die dreidimensionale Oberflächenstruktur wenigstens einen, vorteilhafterweise mehrere, zwischen wenigstens zwei Rippen und/oder Rippenabschnitten der Rippenstruktur ausgebildeten Zwischenraum umfasst. Ein jeweiliger Zwischenraum wird einerseits begrenzt durch die Oberfläche des Oberflächenbereichs und zu einer jeweiligen Seite hin durch die Rippen bzw. Rippenabschnitte. Auf einer im implantierten Zustand dem Knochen zugewandten Seite ist ein jeweiliger Zwischenraum offen, und bildet so für eine zementierte Verankerung des Implantats einen Raum zum Aufnehmen des Zements.

Gemäß einer Ausführungsform ist vorgesehen, dass eine jeweilige Rippe oder ein jeweiliger Rippenabschnitt in der Ebene in Bezug auf eine in der Ebene verlaufenden Bezugsachse längs zu der Bezugsachse, oder quer zu der Bezugsachse oder schräg, insbesondere in einem Winkel größer 0° und kleiner 180°, zu der Bezugsachse oder gekrümmt oder abgewinkelt verläuft.

Gemäß einer Ausführungsform ist vorgesehen, dass wenigstens zwei oder mehr Rippen oder wenigstens zwei oder mehr Rippenabschnitte der Rippenstruktur wenigstens eine Kreuzung, insbesondere in Form einer T-Kreuzung, Y-Kreuzung, +-Kreuzung, Sternkreuzung, ausbilden. Solche Kreuzungen können die Stabilität bzw. Festigkeit der Rippenstruktur erhöhen und damit die Festigkeit des femoralen Implantats verbessern.

Es ist vorgesehen, dass Rippen oder Rippenabschnitte der Rippenstruktur nach einem wabenförmigen Muster angeordnet sind, sodass jeweils sechs Rippen oder Rippenabschnitte eine jeweilige Zelle des wabenförmigen Musters begrenzen. Bei einer gleichmäßigen Wabenform treffen die jeweiligen Rippen oder Rippenabschnitte einer jeweiligen Zelle in einem Winkel von 120° aufeinander. Es sind auch Abweichungen von einer idealen Wabenform denkbar. Alternativ kann auch ein einem wabenförmigen Muster ähnliches Musters ausgebildet werden, beispielsweise können die Zellen auch durch ein Dreieck oder Fünfeck, als durch jeweils drei oder fünf Rippen oder Rippenabschnitte, bzw. sieben oder mehr Rippen oder Rippenabschnitte begrenzt sein. Eine Rippenstruktur, die nach einem solchen Muster, insbesondere einem wabenförmigen Muster ausgebildet ist, weist eine besonders vorteilhafte Festigkeit bzw. Stabilität auf.

Gemäß einer nicht erfindungsgemäßen Ausführungsform ist vorgesehen, dass eine jeweilige Rippe oder ein jeweiliger Rippenabschnitt einen in der Ebene spiralförmigen Verlauf umfasst. In diesem Zusammenhang kann es vorteilhaft sein, wenn der Oberflächenbereich in einem Randbereich durch eine oder mehrere Rippen oder Rippenabschnitt vollständig umrandet wird. Auf diese Weise wird ein Raum zum Aufnehmen von Zement bereitgestellt.

Die eingangs genannte Aufgabe wird auch gelöst durch tiborales Implantatteil für eine orthopädische Kniegelenkprothese nach Anspruch 5. Das tiborale Implantatteil weist wenigstens einen dem Knochen zugewandten und im implantierten Zustand mit dem Knochen in Kontakt stehenden Oberflächenbereich auf. Das tiborale Implantatteil ist wenigstens teilweise, zumindest der mit dem Knochen in Kontakt stehende Oberflächenbereich, aus einem Kunststoff, insbesondere Polyetheretherketon (PEEK) oder Polyetheretherketon, (PEEK),-Verbundwerkstoff, gefertigt. Das tiborale Implantatteil weist eine dreidimensionale Oberflächenstruktur bei dem dem Knochen zugewandten Oberflächenbereich auf, und die dreidimensionale Oberflächenstruktur umfasst wenigstens eine Rippenstruktur, wobei wenigstens ein sich auf einer Oberfläche des Oberflächenbereichs in einer Ebene erstreckender erster Rippenabschnitt oder eine sich auf der Oberfläche des Oberflächenbereichs in der Ebene erstreckende erste Rippe der Rippenstruktur und ein sich auf der Oberfläche des Oberflächenbereichs in der Ebene erstreckender zweiter Rippenabschnitt oder eine sich auf der Oberfläche des Oberflächenbereichs in der Ebene erstreckende zweite Rippe der Rippenstruktur in der Ebene zumindest abschnittsweise in voneinander verschiedene Richtungen verlaufen.

Gemäß weiteren Ausführungsformen ist das tiborale Implantatteil, beziehungsweise der Oberflächenbereich des tiboralen Implantatteils analog zu dem in Bezug auf das femorale Implantatteil beschriebenen Oberflächenbereich ausgebildet.

Es ist beispielsweise vorgesehen, dass die dreidimensionale Oberflächenstruktur wenigstens einen, vorteilhafterweise mehrere, zwischen wenigstens zwei Rippen und/oder Rippenabschnitten der Rippenstruktur ausgebildeten Zwischenraum umfasst.

Gemäß einer Ausführungsform ist vorgesehen, dass eine jeweilige Rippe oder ein jeweiliger Rippenabschnitt in der Ebene in Bezug auf eine in der Ebene verlaufenden Bezugsachse längs zu der Bezugsachse, oder quer zu der Bezugsachse oder schräg, insbesondere in einem Winkel größer 0° und kleiner 180°, zu der Bezugsachse oder gekrümmt oder abgewinkelt verläuft.

Gemäß einer Ausführungsform ist vorgesehen, dass wenigstens zwei oder mehr Rippen oder wenigstens zwei oder mehr Rippenabschnitte der Rippenstruktur wenigstens eine Kreuzung, insbesondere eine T-Kreuzung, Y-Kreuzung, +-Kreuzung, Sternkreuzung, ausbilden.

Es ist vorgesehen, dass Rippen oder Rippenabschnitte der Rippenstruktur nach einem wabenförmigen Muster angeordnet sind, sodass jeweils sechs Rippen oder Rippenabschnitte eine jeweilige Zelle des wabenförmigen Musters begrenzen.

Gemäß einer nicht erfindungsgemäßen Ausführungsform ist vorgesehen, dass eine jeweilige Rippe oder ein jeweiliger Rippenabschnitt einen in der Ebene spiralförmigen Verlauf umfasst. In diesem Zusammenhang kann es vorteilhaft sein, wenn der Oberflächenbereich in einem Randbereich durch eine oder mehrere Rippen oder Rippenabschnitt vollständig umrandet wird.

Die eingangs genannte Aufgabe wird auch gelöst durch eine orthopädische Kniegelenkprothese nach Anspruch 9. Eine solche orthopädische Kniegelenkprothese umfasst ein femorales Implantatteil gemäß den beschriebenen Ausführungsformen und/oder ein tiborales Implantatteil gemäß den beschriebenen Ausführungsformen, und gegebenenfalls ein Meniskus-Ersatzteil, welches einen Gleitpartner für das femorale Implantatteil bildet und an dem tiboralen Implantatteil gehalten ist.

Weitere Ausführungsformen betreffen ein Verfahren zur Herstellung eines femoralen Implantatteils gemäß den beschriebenen Ausführungsformen und/oder eines tiboralen Implantatteils gemäß den beschriebenen Ausführungsformen. Es ist beispielsweise vorgesehen, dass das femorale Implantatteil und/oder das tiborale Implantatteil in einem Spritzgussverfahren oder in einem additiven Fertigungsverfahren hergestellt wird. Bei einem Spritzgussverfahren wird die dreidimensionalen Oberflächenstruktur durch Einspritzen von Material in einen entsprechenden Formhohlraum hergestellt. Bei einem additiven Fertigungsverfahren, 3D-Druck wird die dreidimensionale Oberflächenstruktur durch schichtweises Hinzufügen von Material auf die Oberfläche hergestellt.

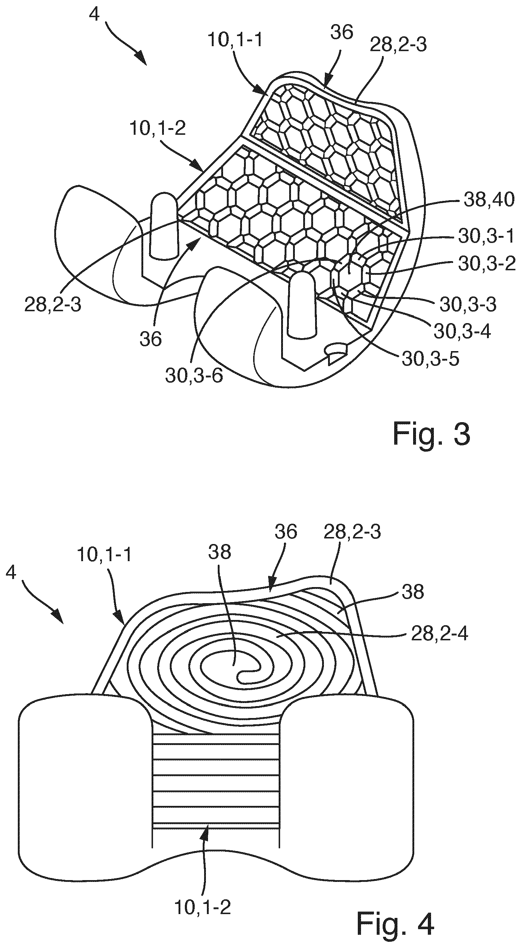

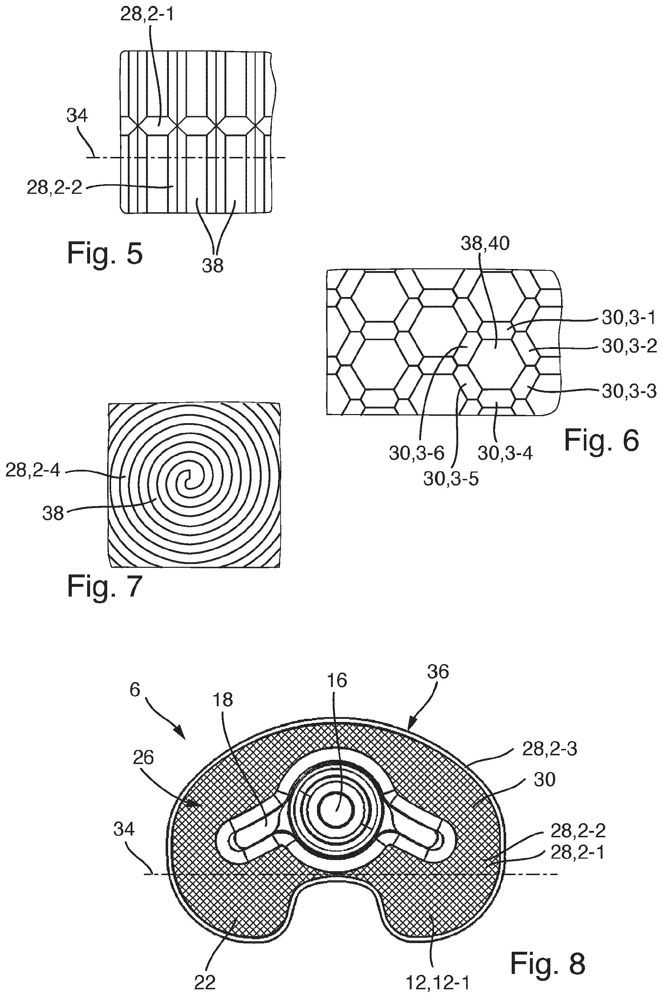

Weitere Merkmale, Einzelheiten und Vorteile der Erfindung ergeben sich aus den beigefügten Patentansprüchen und aus der zeichnerischen Darstellung und nachfolgenden Beschreibung einer erfindungsgemäßen Ausführungsform der orthopädischen Kniegelenksprothese gemäß der Figuren 3 und 6 . Dabei bezeichnen gleiche Bezugszeichen in verschiedenen Figuren jeweils gleiche oder zumindest ihrer Funktion nach vergleichbare Elemente. Bei der Beschreibung einzelner Figuren wird gegebenenfalls auch auf Elemente aus anderen Figuren Bezug genommen.

In der Zeichnung zeigt:
- Fig. 1: eine explosionsartige Darstellung einer erfindungsgemäßen Ausführungsform einer orthopädischen Kniegelenksprothese mit einem femoralen Implantatteil, einem tiboralen Implantatteil und einem an dem tiboralen Implantatteil festlegbaren Meniskus-Ersatzteil;
- Fig. 2: eine Ansicht eines femoralen Implantatteils für eine orthopädische Kniegelenksprothese gemäß einer nicht erfindungsgemäßen Ausführungsform;
- Fig. 3: eine Ansicht eines femoralen Implantatteils für eine orthopädische Kniegelenksprothese gemäß einer weiteren Ausführungsform;
- Fig. 4: eine Ansicht eines femoralen Implantatteils für eine orthopädische Kniegelenksprothese gemäß einer weiteren nicht erfindungsgemäßen Ausführungsform;
- Fig. 5: eine Ansicht einer dreidimensionalen Oberflächenstruktur gemäß einer ersten, nicht erfindungsgemäßen Ausführungsform;
- Fig. 6: eine Ansicht einer dreidimensionalen Oberflächenstruktur gemäß einer weiteren Ausführungsform;
- Fig. 7: eine Ansicht einer dreidimensionalen Oberflächenstruktur gemäß einer weiteren nicht erfindungsgemäßen Ausführungsform;
- **Fig.** 8: eine Ansicht eines tiboralen Implantatteils in Draufsicht von unten.

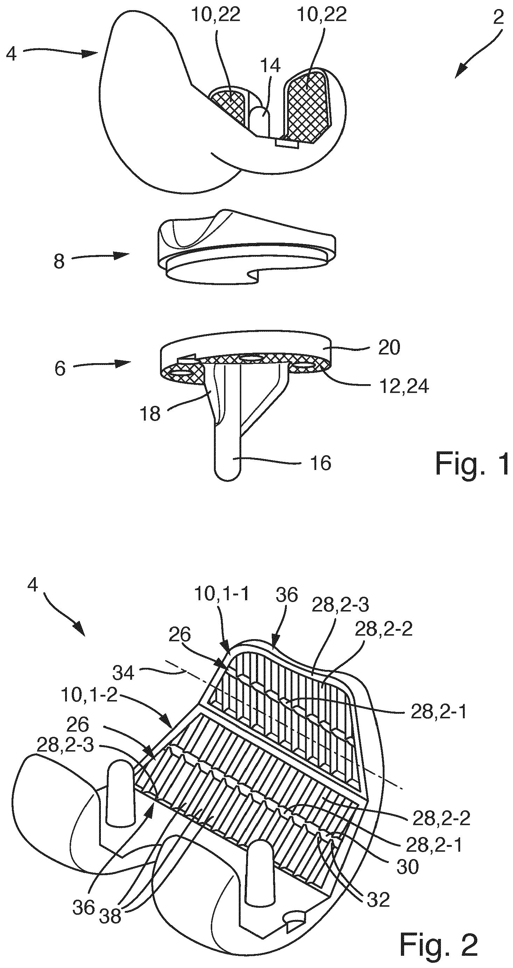

Figur 1 zeigt in explosionsartiger Darstellung eine insgesamt mit dem Bezugszeichen 2 bezeichnete orthopädische Kniegelenkprothese mit einem femoralen Implantatteil **4,** einem tiboralen Implantatteil 6 und einem Meniskus-Ersatzteil **8,** welches typischerweise an dem tiboralen Implantatteil 6 verankerbar **ist.** Das femorale Implantatteil 4 umfasst einen einem nicht dargestellten Knochen des Oberschenkels (Femur) zugewandten und mit dem Knochen in Kontakt stehenden Oberflächenbereich 10.

Das tiborale Implantatteil 6 weist ebenfalls einen dem Knochen des Unterschenkels (Tibia) zugewandten und mit dem Knochen in Kontakt stehenden Oberflächenbereich 12 **auf.**

Das femorale Implantatteil 4 weist zumindest einen dom- oder schaftförmigen Vorsprung 14 und das tiborale Implantatteil 6 einen langgestreckten Vorsprung 16 auf, die jeweils zum Eingriff und zur Verankerung an dem Oberschenkel- bzw. Unterschenkelknochen dienen. Der Vorsprung 16 des tiboralen Implantatteils 6 ist zudem über in der Vertikalebene erstreckte wangenförmige bzw. flügelförmige Abschnitte 18 an einem scheibenförmigen Plateauteil 20 des tiboralen Implantatteils 6 angebunden.

Im beispielhaft und bevorzugt dargestellten Fall ist das femorale Implantatteil 4 wie auch das tiborale Implantatteil 6 im Bereich ihres Knochenkontakts, nämlich Oberflächenbereich 10, 12 jeweils mit einer dreidimensionalen Oberflächenstruktur 22 bzw. 24 ausgebildet. In beiden Fällen ragt diese dreidimensionale Oberflächenstruktur 22, 24 makroskopisch über wenigstens 0,5 mm und insbesondere bis 3 mm von einer Oberfläche des jeweiligen Oberflächenbereich 10, 12 des jeweilige Implantatteil 4 bzw. 6 hervor.

Unter Bezugnahme auf die Figuren 2 bis 7 werden am Beispiel des femoralen Implantatteils 4 im Folgenden verschiedene vorteilhafte Ausführungsformen der dreidimensionalen Oberflächenstruktur 22 erläutert. Lediglich die Figuren 3 und 6 zeigen hierbei erfindungsgemäße Ausgestaltungen.

Die dreidimensionale Oberflächenstruktur 24 des tiboralen Implantatteils 6 kann entsprechend analog ausgebildet sein.

Fig. 2 zeigt ein femorales Implantatteil 4. Im Beispiel umfasst das femorale Implantatteil 4 mehrere mit dem Knochen in Kontakt stehenden Oberflächenbereiche 10 bei denen eine dreidimensionalen Oberflächenstruktur 14 ausgebildet ist. Im Beispiel werden die Oberflächenbereiche 10-1, und 10-2 betrachtet. Weitere Oberflächenbereiche, beispielsweise im Bereich der Femurkondylen, sind aufgrund der dargestellten Ansicht, nicht einsehbar. Auf den weiteren Oberflächenbereichen können auch dreidimensionale Oberflächenstrukturen (nicht dargestellt) ausgebildet sein.

Eine jeweilige Oberflächenstruktur 14 der Oberflächenbereiche 10-1, und 10-2 umfasst eine Rippenstruktur 26. Eine solche Rippenstruktur umfasst Rippen 28 bzw. Rippenabschnitte 30. Unter einer Rippe 28 wird beispielsweise eine ohne Unterbrechung verlaufende, ausgehend von der Oberfläche ausgewölbte stegförmige Struktur, verstanden. Ein Rippenabschnitt 30 ist beispielsweise ein beliebiger Abschnitt einer solchen Rippe 28. Insbesondere ist ein Rippenabschnitt 30 ein von einer Kreuzung 32, an der sich wenigstens zwei Rippen 18 kreuzen bzw., aufeinandertreffen, bis zu einer weiteren Kreuzung 32 verlaufender Abschnitt.

In der Ausführungsform gemäß Fig. 2 umfasst die Rippenstruktur 26 der Oberflächenbereiche 10-1, und 10-2 längs zu einer Bezugsachse 34 verlaufende Rippen 28-1, und quer zu der Bezugsachse 34 verlaufende Rippen 28-2. Beide Oberflächenbereiche 10-1, und 10-2 werden in einem jeweiligen Randbereich 36 durch eine umlaufende Rippe 28-3 vollständig umrandet.

Alternativ können Rippen 28 bzw. Rippenabschnitte 30 auch schräg zu der Bezugsachse 34 verlaufen.

Im dargestellten Beispiel gemäß Fig. 2 treffen längs und quer verlaufende Rippen 28-1, 28-2 an Kreuzungen 22 in einem rechten Winkel aufeinander. Die dargestellten Kreuzungen sind daher von der Form einer +-Kreuzung. Alternativ können Rippen 28 auch in beliebigen anderen Winkeln oder in einer anderen Anzahl sich kreuzen bzw. aufeinandertreffen. Weiter mögliche Kreuzungsformen sind beispielsweise T-Kreuzung, Y-Kreuzung, Sternkreuzung oder beliebig andere Kreuzungsformen.

Im dargestellten Beispiel gemäß Fig. 2 sind zwischen wenigstens Rippen 28, 28-1, 28-2, 28-3 bzw. Rippenabschnitten 30 der Rippenstruktur 26 mehrere Zwischenräume 38 ausgebildet. Ein jeweiliger Zwischenraum 38 wird einerseits begrenzt durch die Oberfläche des Oberflächenbereichs 10, 10-1, 10-2 und zu einer jeweiligen Seite hin durch die Rippen 28, 28-1, 28-2, 28-3 bzw. Rippenabschnitte 30. Auf einer im implantierten Zustand dem Knochen zugewandten Seite ist ein jeweiliger Zwischenraum 38 offen.

Fig. 3 zeigt eine weitere Ausführungsform. Im Beispiel werden die Oberflächenbereiche 10-1, und 10-2 betrachtet. Eine jeweilige Oberflächenstruktur 14 der Oberflächenbereiche 10-1, und 10-2 umfasst eine Rippenstruktur 26. Die Oberflächenstruktur 14 umfasst Rippen 28, bzw. Rippenabschnitte 30, die nach einem wabenförmigen Muster angeordnet sind. Im Beispiel begrenzen sechs Rippenabschnitte 30-1, 30-2, 30-3, 30-4, 30-5, 30-6 eine jeweilige Zelle 40 des wabenförmigen Musters.

Eine jeweilige Zelle 40 des wabenförmigen Musters bildet einen jeweiligen Zwischenraum 38.

Fig. 4 zeigt eine weitere Ausführungsform. Im Beispiel werden die Oberflächenbereiche 10-1, und 10-2 betrachtet. Eine jeweilige Oberflächenstruktur 14 der Oberflächenbereiche 10-1, und 10-2 umfasst eine Rippenstruktur 26.

Die Rippenstruktur 26 des Oberflächenbereichs 10-1 umfasst eine Rippe 28-4 mit einem spiralförmigen Verlauf. Die Rippe 28-4 umfasst mehrere Rippenabschnitte 30, die aufgrund des spiralförmigen Verlaufs in der Ebene zumindest abschnittsweise in voneinander verschiedene Richtungen verlaufen. Der Oberflächenbereich 10-1 wird in einem Randbereich 36 durch eine umlaufende Rippe 28-3 vollständig umrandet.

Die Rippenstruktur 26 des Oberflächenbereichs 10-1 umfasst mehrere längs zu einer Bezugsachse 34 verlaufende Rippen 28-1.

Der Oberflächenbereich 10-2 wird in einem Randbereich 36 ebenfalls durch eine umlaufende Rippe 28-3 vollständig umrandet.

In den Figuren 5, 6, 7 sind Ausschnitte der dreidimensionalen Oberflächenstrukturen aus Figuren 2, 3, 4 dargestellt.

Grundsätzlich sind bei einem femoralen Implantat 4 Kombination der dargestellten oder auch weiteren dreidimensionalen Oberflächenstrukturen in verschiedenen Oberflächenbereich möglich. Es ist auch eine Kombination innerhalb eines jeweiligen Oberflächenbereichs möglich. Eine jeweilige Oberflächenstruktur bzw. eine jeweilige Anordnung der Rippen bzw. Rippenabschnitte bzw. ein jeweiliges Muster muss sich bei einem jeweiligen Oberflächenbereich nicht zwingend über einen gesamten Oberflächenbereich erstrecken.

Fig. 8 zeigt ein tiborales Implantat 8 in einer Ansicht von unten auf den im implantierten Zustand mit dem Knochen in Kontakt stehenden Oberflächenbereich 12. Gemäß Fig. 8 umfasst das tiborale Implantat 8 einen durchgehenden Oberflächenbereich 12. Der Oberflächenbereich 12 kann aber auch in mehrere einzelne Oberflächenbereiche 12 unterteilt sein. Der Oberflächenbereich 12 kann eine Oberflächenstruktur umfassen, die analog zu den in Fig. 2 bis 7 dargestellten oder auch weiteren dreidimensionalen Oberflächenstrukturen ausgebildet ist. Im Beispiel umfasst die Oberflächenstruktur eine Rippenstruktur 26, mit Rippen 28, 28-1, 28-2 und Rippenabschnitten 30, die schräg in Bezug auf eine Bezugsachse 34 verlaufen. Weiter ist in einem Randbereich 36 eine vollständige Umrandung durch eine umlaufende Rippe 28-3 vorgesehen.

## Patentansprüche

1. Femorales Implantatteil (4) für eine orthopädische Kniegelenkprothese (2), wobei das femorale Implantatteil (4) wenigstens einen dem Knochen zugewandten und im implantierten Zustand mit dem Knochen in Kontakt stehenden Oberflächenbereich (10) aufweist, wobei das femorale Implantatteil (4) wenigstens teilweise, zumindest der mit dem Knochen in Kontakt stehende Oberflächenbereich (10), aus einem Kunststoff, insbesondere Polyetheretherketon oder Polyetheretherketon -Verbundwerkstoff, gefertigt ist, und wobei das femorale Implantatteil (4) eine dreidimensionale Oberflächenstruktur (14) bei dem dem Knochen zugewandten Oberflächenbereich (10) aufweist, und die dreidimensionale Oberflächenstruktur (14) wenigstens eine Rippenstruktur (26) umfasst, wobei wenigstens ein sich auf einer Oberfläche des Oberflächenbereichs (10) in einer Ebene erstreckender erster Rippenabschnitt (30) oder eine sich auf der Oberfläche des Oberflächenbereichs (10) in der Ebene erstreckende erste Rippe (28, 28-1) der Rippenstruktur (26) und ein sich auf der Oberfläche des Oberflächenbereichs (10) in der Ebene erstreckender zweiter Rippenabschnitt (30) oder eine sich auf der Oberfläche des Oberflächenbereichs (10) in der Ebene erstreckende zweite Rippe (28, 28-2) der Rippenstruktur (26) in der Ebene zumindest abschnittsweise in voneinander verschiedene Richtungen verlaufen, **dadurch gekennzeichnet, dass** die Rippen (28) oder Rippenabschnitte (30) der Rippenstruktur (26) nach einem wabenförmigen Muster angeordnet sind, sodass jeweils sechs Rippen (28) oder Rippenabschnitte (30, 30-1, 30-2, 30-3, 30-4, 30-5, 30-6) eine jeweilige Zelle (40) des wabenförmigen Musters begrenzen.

2. Femorales Implantatteil (4) nach Anspruch 1, wobei die dreidimensionale Oberflächenstruktur (14) wenigstens einen zwischen wenigstens zwei Rippen (28, 28-1, 28-2, 28-3) und/oder Rippenabschnitten (30) der Rippenstruktur (26) ausgebildeten Zwischenraum (38) umfasst.

3. Femorales Implantatteil (4) nach einem der Ansprüche 1 oder 2, wobei eine jeweilige Rippe (28, 28-1, 28-2, 28-3) oder ein jeweiliger Rippenabschnitt (30) in der Ebene in Bezug auf eine in der Ebene verlaufenden Bezugsachse (34) längs zu der Bezugsachse (34), oder quer zu der Bezugsachse (34) oder schräg, insbesondere in einem Winkel größer 0° und kleiner 180°, zu der Bezugsachse (34) oder gekrümmt oder abgewinkelt verläuft.

4. Femorales Implantatteil (4) nach einem der vorhergehenden Ansprüche, wobei wenigstens zwei oder mehr Rippen (28, 28-1, 28-2, 28-3) oder wenigstens zwei oder mehr Rippenabschnitte (30) der Rippenstruktur (26) wenigstens eine Kreuzung (32), insbesondere eine T-Kreuzung, Y-Kreuzung, +-Kreuzung, Sternkreuzung, ausbilden.

5. Tiborales Implantatteil (6) für eine orthopädische Kniegelenkprothese (2), wobei das tiborale Implantatteil (6) wenigstens einen dem Knochen zugewandten und im implantierten Zustand mit dem Knochen in Kontakt stehenden Oberflächenbereich (12) aufweist, wobei das tiborale Implantatteil (6) wenigstens teilweise, zumindest der mit dem Knochen in Kontakt stehende Oberflächenbereich (12), aus einem Kunststoff, insbesondere Polyetheretherketon oder Polyetheretherketon -Verbundwerkstoff, gefertigt ist, und wobei das tiborale Implantatteil (6) eine dreidimensionale Oberflächenstruktur (14) bei dem dem Knochen zugewandten Oberflächenbereich (12) aufweist, und die dreidimensionale Oberflächenstruktur wenigstens eine Rippenstruktur (26) umfasst, wobei wenigstens ein sich auf einer Oberfläche des Oberflächenbereichs (12) in einer Ebene erstreckender erster Rippenabschnitt (30) oder eine sich auf der Oberfläche des Oberflächenbereichs (12) in der Ebene erstreckende erste Rippe (28, 28-1) der Rippenstruktur (26) und ein sich auf der Oberfläche des Oberflächenbereichs (12) in der Ebene erstreckender zweiter Rippenabschnitt (30) oder eine sich auf der Oberfläche des Oberflächenbereichs (12) in der Ebene erstreckende zweite Rippe (28, 28-2) der Rippenstruktur (26) in der Ebene zumindest abschnittsweise in voneinander verschiedene Richtungen verlaufen, **dadurch gekennzeichnet, dass** Rippen (28) oder Rippenabschnitte (30) der Rippenstruktur (26) nach einem wabenförmigen Muster angeordnet sind, sodass jeweils sechs Rippen (28) oder Rippenabschnitte (30, 30-1, 30-2, 30-3, 30-4, 30-5, 30-6) eine jeweilige Zelle (40) des wabenförmigen Musters begrenzen.

6. Tiborales Implantatteil (6) nach Anspruch 5, wobei die dreidimensionale Oberflächenstruktur (14) wenigstens einen zwischen wenigstens zwei Rippen (28, 28-1, 28-2, 28-3) und/oder Rippenabschnitten (30) der Rippenstruktur (26) ausgebildeten Zwischenraum (38) umfasst

7. Tiborales Implantatteil (6) nach einem der Ansprüche 5 oder 6, wobei eine jeweilige Rippe (28, 28-1, 28-2, 28-3) oder ein jeweiliger Rippenabschnitt (30) in der Ebene in Bezug auf eine in der Ebene verlaufenden Bezugsachse (34) längs zu der Bezugsachse (34), oder quer zu der Bezugsachse (34) oder schräg, insbesondere in einem Winkel größer 0° und kleiner 180°, zu der Bezugsachse (34) oder gekrümmt oder abgewinkelt verläuft.

8. Tiborales Implantatteil (6) nach einem der Ansprüche 5 bis 7, wobei wenigstens zwei oder mehr Rippen (28, 28-1, 28-2, 28-3) oder wenigstens zwei oder mehr Rippenabschnitte (30) der Rippenstruktur (26) wenigstens eine Kreuzung (32), insbesondere eine T-Kreuzung, Y-Kreuzung, +-Kreuzung, Sternkreuzung, ausbilden.

9. Orthopädische Kniegelenkprothese (2) umfassend ein femorales Implantatteil (4), nach einem der Ansprüche 1 bis 4, und ein tiborales Implantatteil (6) nach einem der Ansprüche 5 bis 8, und gegebenenfalls mit einem Meniskus-Ersatzteil (8), welches einen Gleitpartner für das femorale Implantatteil (4) bildet und an dem tiboralen Implantatteil (6) gehalten ist.

10. Verfahren zur Herstellung eines femoralen Implantatteils (4) nach einem der Ansprüche 1 bis 4 und/oder eines tiboralen Implantatteils (6) nach einem der Ansprüche 5 bis 8, wobei das femorale Implantatteil (4) und/oder das tiborale Implantatteil (6) in einem Spritzgussverfahren oder in einem additiven Fertigungsverfahren hergestellt wird.

## Claims

1. A femoral implant part (4) for an orthopedic knee joint prosthesis (2), wherein the femoral implant part (4) has at least one surface region (10) which faces the bone and in the implanted state is in contact with the bone, wherein at least part of the femoral implant part (4), at least the surface region (10) in contact with the bone, is made from a plastics material, in particular polyether ether ketone or polyether ether ketone composite material, and wherein the femoral implant part (4) has a three-dimensional surface structure (14) in the surface region (10) facing the bone, and the three-dimensional surface structure (14) comprises at least one rib structure (26), wherein at least a first rib portion (30) extending on a surface of the surface region (10) in a plane or a first rib (28, 28-1) of the rib structure (26) extending on the surface of the surface region (10) in the plane and a second rib portion (30) extending on the surface of the surface region (10) in the plane or a second rib (28, 28-2) of the rib structure (26) extending on the surface of the surface region (10) in the plane extend in the plane in mutually different directions, at least in portions, **characterized in that** the ribs (28) or rib portions (30) of the rib structure (26) are arranged in a honeycomb pattern, with the result that in each case six ribs (28) or rib portions (30, 30-1, 30-2, 30-3, 30-4, 30-5, 30-6) delimit a particular cell (40) of the honeycomb pattern.

2. The femoral implant part (4) according to claim 1, wherein the three-dimensional surface structure (14) comprises at least one intermediate space (38) formed between at least two ribs (28, 28-1, 28-2, 28-3) and/or rib portions (30) of the rib structure (26).

3. The femoral implant part (4) according to either one of claims 1 or 2, wherein, with respect to a reference axis (34) extending in the plane, a particular rib (28, 28-1, 28-2, 28-3) or a particular rib portion (30) extends in the plane longitudinally with respect to the reference axis (34), or transversely with respect to the reference axis (34), or obliquely, in particular at an angle of greater than 0° and less than 180°, with respect to the reference axis (34), or curved or at an angle.

4. The femoral implant part (4) according to any one of the preceding claims, wherein at least two or more ribs (28, 28-1, 28-2, 28-3) or at least two or more rib portions (30) of the rib structure (26) form at least one intersection (32), in particular a T-intersection, Y-intersection, +-intersection, or star intersection.

5. A tibial implant part (6) for an orthopedic knee joint prosthesis (2), wherein the tibial implant part (6) has at least one surface region (12) which faces the bone and in the implanted state is in contact with the bone, wherein at least part of the tibial implant part (6), at least the surface region (12) in contact with the bone, is made from a plastics material, in particular polyether ether ketone or polyether ether ketone composite material, and wherein the tibial implant part (6) has a three-dimensional surface structure (14) in the surface region (12) facing the bone, and the three-dimensional surface structure comprises at least one rib structure (26), wherein at least a first rib portion (30) extending on a surface of the surface region (12) in a plane or a first rib (28, 28-1) of the rib structure (26) extending on the surface of the surface region (12) in the plane and a second rib portion (30) extending on the surface of the surface region (12) in the plane or a second rib (28, 28-2) of the rib structure (26) extending on the surface of the surface region (12) in the plane extend in the plane in mutually different directions, at least in portions, **characterized in that** ribs (28) or rib portions (30) of the rib structure (26) are arranged in a honeycomb pattern, with the result that in each case six ribs (28) or rib portions (30, 30-1, 30-2, 30-3, 30-4, 30-5, 30-6) delimit a particular cell (40) of the honeycomb pattern.

6. The tibial implant part (6) according to claim 5, wherein the three-dimensional surface structure (14) comprises at least one intermediate space (38) formed between at least two ribs (28, 28-1, 28-2, 28-3) and/or rib portions (30) of the rib structure (26).

7. The tibial implant part (6) according to either one of claims 5 or 6, wherein, with respect to a reference axis (34) extending in the plane, a particular rib (28, 28-1, 28-2, 28-3) or a particular rib portion (30) extends in the plane longitudinally with respect to the reference axis (34), or transversely with respect to the reference axis (34), or obliquely, in particular at an angle of greater than 0° and less than 180°, with respect to the reference axis (34), or curved or at an angle.

8. The tibial implant part (6) according to any one of claims 5 to 7, wherein at least two or more ribs (28, 28-1, 28-2, 28-3) or at least two or more rib portions (30) of the rib structure (26) form at least one intersection (32), in particular a T-intersection, Y-intersection, +-intersection, or star intersection.

9. An orthopedic knee joint prosthesis (2) comprising a femoral implant part (4) according to any one of claims 1 to 4, and a tibial implant part (6) according to any one of claims 5 to 8, and optionally also having a meniscus replacement part (8) which forms a sliding partner for the femoral implant part (4) and is held on the tibial implant part (6).

10. A method for producing a femoral implant part (4) according to any one of claims 1 to 4 and/or a tibial implant part (6) according to any one of claims 5 to 8, wherein the femoral implant part (4) and/or the tibial implant part (6) is produced in an injection molding process or in an additive manufacturing process.

## Revendications

1. élément d'implant fémoral (4) pour une prothèse orthopédique du genou (2), l'élément d'implant fémoral (4) présentant au moins une zone de surface (10) tournée vers l'os et, en état implanté, en contact avec l'os, l'élément d'implant fémoral (4) étant au moins partiellement, au minimum la zone de surface (10) en contact avec l'os, constitué d'un plastique, en particulier de polyétheréthercétone ou d'un matériau composite à base de polyétheréthercétone, fabriqué
et dans lequel l'élément d'implant fémoral (4) présente une structure de surface tridimensionnelle (14) au niveau de la zone de surface (10) tournée vers l'os, ladite structure de surface tridimensionnelle (14) comprenant au moins une structure à nervures (26), dans laquelle au moins un premier segment de nervure (30) s'étendant dans un plan sur une surface de la zone de surface (10) ou une première nervure (28, 28-1) s'étendant dans le plan sur la surface de la zone de surface (10) de la structure à nervures (26), et un deuxième segment de nervure (30) s'étendant dans le plan sur la surface de la zone de surface (10) ou une deuxième nervure (28, 28-2) s'étendant dans le plan sur la surface de la zone de surface (10) de la structure à nervures (26), s'étendent au moins partiellement dans des directions différentes l'une de l'autre dans le plan, **caractérisé en ce que** les nervures (28) ou segments de nervure (30) de la structure à nervures (26) sont disposés selon un motif en nid d'abeille, de sorte que six nervures (28) ou segments de nervure (30, 30-1, 30-2, 30-3, 30-4, 30-5, 30-6) délimitent chacune une cellule (40) du motif en nid d'abeille.

2. élément d'implant fémoral (4) selon la revendication 1, dans lequel la structure de surface tridimensionnelle (14) comprend au moins un espace (38) formé entre au moins deux nervures (28, 28-1, 28-2, 28-3) et/ou segments de nervure (30) de la structure à nervures (26).

3. élément d'implant fémoral (4) selon l'une des revendications 1 ou 2, dans lequel chaque nervure (28, 28-1, 28-2, 28-3) ou chaque segment de nervure (30) s'étend dans le plan par rapport à un axe de référence (34) courant dans le plan soit parallèlement à l'axe de référence (34), soit perpendiculairement à l'axe de référence (34), soit obliquement, en particulier selon un angle supérieur à 0° et inférieur à 180°, par rapport à l'axe de référence (34), ou est courbée ou coudée.

4. élément d'implant fémoral (4) selon l'une quelconque des revendications précédentes, dans lequel au moins deux ou plusieurs nervures (28, 28-1, 28-2, 28-3) ou au moins deux ou plusieurs segments de nervure (30) de la structure à nervures (26) forment au moins une intersection (32), en particulier une intersection en T, en Y, en +, ou en étoile.

5. élément d'implant tibial (6) pour une prothèse orthopédique du genou (2), l'élément d'implant tibial (6) présentant au moins une zone de surface (12) tournée vers l'os et, en état implanté, en contact avec l'os, l'élément d'implant tibial (6) étant au moins partiellement, au minimum la zone de surface (12) en contact avec l'os, constitué d'un plastique, en particulier de polyétheréthercétone ou d'un matériau composite à base de polyétheréthercétone, fabriqué
et dans lequel l'élément d'implant tibial (6) présente une structure de surface tridimensionnelle (14) au niveau de la zone de surface (12) tournée vers l'os, ladite structure de surface tridimensionnelle (14) comprenant au moins une structure à nervures (26), dans laquelle au moins un premier segment de nervure (30) s'étendant dans un plan sur une surface de la zone de surface (12) ou une première nervure (28, 28-1) s'étendant dans le plan sur la surface de la zone de surface (12) de la structure à nervures (26), et un deuxième segment de nervure (30) s'étendant dans le plan sur la surface de la zone de surface (12) ou une deuxième nervure (28, 28-2) s'étendant dans le plan sur la surface de la zone de surface (12) de la structure à nervures (26), s'étendent au moins partiellement dans des directions différentes l'une de l'autre dans le plan, **caractérisé en ce que** les nervures (28) ou segments de nervure (30) de la structure à nervures (26) sont disposés selon un motif en nid d'abeille, de sorte que six nervures (28) ou segments de nervure (30, 30-1, 30-2, 30-3, 30-4, 30-5, 30-6) délimitent chacune une cellule (40) du motif en nid d'abeille.

6. élément d'implant tibial (6) selon la revendication 5, dans lequel la structure de surface tridimensionnelle (14) comprend au moins un espace (38) formé entre au moins deux nervures (28, 28-1, 28-2, 28-3) et/ou segments de nervure (30) de la structure à nervures (26)

7. élément d'implant tibial (6) selon l'une des revendications 5 ou 6, dans lequel chaque nervure (28, 28-1, 28-2, 28-3) ou chaque segment de nervure (30) s'étend dans le plan par rapport à un axe de référence (34) courant dans le plan soit parallèlement à l'axe de référence (34), soit perpendiculairement à l'axe de référence (34), soit obliquement, en particulier selon un angle supérieur à 0° et inférieur à 180°, par rapport à l'axe de référence (34), ou est courbée ou coudée.

8. élément d'implant tibial (6) selon l'une quelconque des revendications 5 à 7, dans lequel au moins deux ou plusieurs nervures (28, 28-1, 28-2, 28-3) ou au moins deux ou plusieurs segments de nervure (30) de la structure à nervures (26) forment au moins une intersection (32), en particulier une intersection en T, en Y, en +, ou en étoile.

9. Prothèse orthopédique du genou (2) comprenant un élément d'implant fémoral (4), selon l'une des revendications 1 à 4, et un élément d'implant tibial (6) selon l'une des revendications 5 à 8, et éventuellement un élément de remplacement du ménisque (8) formant un partenaire de glissement pour l'élément d'implant fémoral (4) et maintenu sur l'élément d'implant tibial (6).

10. Procédé de fabrication d'un élément d'implant fémoral (4) selon l'une des revendications 1 à 4 et/ou d'un élément d'implant tibial (6) selon l'une des revendications 5 à 8, dans lequel l'élément d'implant fémoral (4) et/ou l'élément d'implant tibial (6) est fabriqué soit par moulage par injection, soit par un procédé de fabrication additive.
